# EUROPEAN PATENT SPECIFICATION

(11) **EP 3 871 641 B1**
(45) Date of publication and mention of the grant of the patent: **02.07.2025**
(21) Application number: 19876192.6
(22) Date of filing: 22.10.2019
(51) Int. Cl.: A61F 5/56

(54) **INTRAORAL FERRULE WITH ARCH FOR PREVENTING SNORING AND APNOEA**
INTRAORALE FERRULE MIT BOGEN ZUR VERHINDERUNG VON SCHNARCHEN UND APNOE
GOUTTIÈRE INTRABUCCALE AVEC ARC POUR ÉVITER LES RONFLEMENTS ET LES APNÉES

(30) Priority: 24.10.2018 ES 201831605 U
(43) Date of publication of application: 01.09.2021
(73) Proprietor: Navarro Segura, Miguel, 03660 Novelda - Alicante (ES); Mangada Martinez, Federico Juan Carlos, 03540 Alicante (ES)
(72) Inventor: NAVARRO SEGURA, Miguel, 03660 Novelda - Alicante (ES)
(74) Representative: Urizar Anasagasti, Jesus Maria
(86) International application number: PCT/ES2019/070716
(87) International publication number: WO 2020/084183

(56) References cited:
- EP-A1- 2 380 533
- WO-A1-2006/063403
- WO-A2-2011/156396
- FR-A1- 3 041 242
- US-A1- 2010 294 283
- US-A1- 2015 182 373

## Description

### Object of the invention

The purpose of the present invention is to present a new intraoral ferrule with a large arch which arises from the inner anterior region of the ferrule to the posterior part for preventing snoring and apnea, which, being placed in the user's maxilla, prevents the possible displacement of the tongue towards the posterior part of said user's buccal cavity, thereby preventing the possible obstruction of the airway due to the overlap of the posterior part of the tongue, or soft palate, on the posterior pharyngeal wall.

It has a special application in the sector of otorhinolaryngology, where there is a need to correct these problems.

### Background of the invention

Up until now, various devices are reported in the current state of the art for solving problems with snoring and apnea, with specific technical features for each case.

Document EP0312368 comprises an intraoral device for preventing snoring. This device consists of a U-shaped mouthpiece which is shaped to the user's upper dental arching and includes an inclined lower ramp in order to interlock with the mandibular dentition. Normal movements of the mouth, such as closing the jaws, will cause part of the mandibular dentition to interlock with the lower part of the ramp, pushing the lower jaw forward by a cam in order to increase the separation between the base of the tongue and the posterior wall of the pharynx.

Said device is clearly different from the one described in the present Invention.

The Patent ES 2217284 T3, which discloses the features of the preamble of claims 1 and 4, describes a device for preventing stertorous breathing or snoring and adapted to be fastened in the upper part of a person's mouth, comprising a non-rigid and flexible transverse bridge for fastening in the upper part of a person's mouth, preferably in the rear part of the upper part of the mouth, and a member for grasping the tongue, which extends downward from the non-rigid and flexible transverse bridge.

The main difference with respect to said Patent is that instead of grasping the tongue by default and in a fixed manner, in the present invention, a posterior arch is available, just so that in case of a possible displacement of the tongue, the movement thereof can be limited, and, therefore, it can be maintained in a position wherein the airway is not obstructed due to the overlap of the posterior part of the tongue, or soft palate, on the posterior pharyngeal wall.

It should also be commented that our invention is based on an arch which starts from the anterior inner region, not on a transverse bridge.

Patent ES 2118365 T3 defines a dental apparatus for the treatment of snoring episodes and obstructive sleep apnea. This apparatus, made to be worn by a patient when sleeping, has an upper member which is shaped to the patient's upper maxillary dentition, a lower member which is shaped to the patient's mandibular dentition, and a connector means for releasably coupling said upper and lower members to each other, wherein said connector means adjustably maintains said lower member in an anterior position, projected in relation to said upper member, and wherein said connector means enable the lateral movement of said lower member in relation to said upper member in said projected position.

This dental apparatus differs from the present invention, since it is essentially based on positioning the upper member which is shaped to the patient's upper maxillary dentition, with respect to the lower member which is shaped to the patient's lower maxillary dentition by means of a connector which positions said members and, therefore, enables said positions to be adjusted to each other.

Spanish Utility Model U200800413 (ES1067430 U) describes an intraoral device for preventing snoring and apnea. Said device is composed of a body, which fits into the user's maxilla, and exerts uniform pressure thereon, said body incorporating a fin in the posterior part thereof which can vary in shape and size according to the morphology of the user's mouth.

The differences with respect to the present invention are significant, due to the fact that it is a palate, which has a fin, which is stuck in a certain point of the tongue, causing long-term damage to it, in addition to making correct salivation difficult.

Although the dental apparatuses of the state of the art have proven effective for maintaining the jaw in a projected position in order to improve the evidence of the airway, they sometimes result in undesirable side effects. One of the most common side effects is damage to the temporomandibular joint and related jaw muscles and ligaments, especially in individuals who have a tendency to grind their teeth as they sleep. Damage to the temporomandibular joint has been associated with a wide variety of physical illnesses, including migraine headaches. Therefore, many individuals suffering from snoring and sleep apnea disorders are unable to tolerate existing anti-snoring dental apparatuses for long periods of time.

Document US2010294283 A1 describes a system for maintaining a position of a subject's tongue with respect to the mouth comprising a mouth device retractably coupled to a cover that is fixed to an anterior part of the tongue and once placed inside from a subject's mouth, the apparatus can be held in a closed configuration while the subject's mouth remains closed. However, when the subject sleeps and the tongue becomes hypotonic, the subject's mouth normally opens during an episode of sleep disturbance and the base of the tongue collapses backwards obstructing their airway. As the subject's mouth begins to open, the oral apparatus is actuated by the gap between the jaws, causing the sheath attached to the tongue to move relative to the subject's jaw, retracting or pulling it anteriorly..

Document WO2011156396 A2 describes a ferrule that includes a retaining member of the tongue that extends from one side of the tongue to another, in order to limit the movement of the tongue towards the user's throat when the user is exercising or sleeping to keep an air passage open. The tongue retaining member is positioned in said ferrule such that in use it contacts above the tongue in an area behind the second molars and in front of the pharyngeal reflex region of the tongue. The retention element is shaped such that when placed in a user's mouth, the rear region extends above the user's tongue to rest thereon, while the front region extends below the user's tongue.

Document FR3041242 A1 describes an intraoral device for positioning the tongue, comprising a support (ferrule) adapted to be fixed in position with respect to the maxillary dental arch of a patient. This device includes a holder-mounted blade that extends in a longitudinal direction capable of inscribing in a sagittal plane of the patient from a posterior end toward an anterior end. Advantageously, said blade comprises a segment extending from the back to the front and having a component oriented in a sagittal plane and directed beyond the bite plane; so that the blade of the invention allows the tip of the tongue to be deflected upwards and in front of the palate, also ensuring its position during swallowing. This blade is meant to guide the tip of the tongue into the high position, as well as force it to stay there. If the tongue is not correctly placed in the high position, the elastically deformable part causes a forced position of the blade which then pushes the tongue back to the correct position, thus returning to its rest position. Advantageously, the tip of the tongue rests on the blade. The device allows the correct positioning of the tongue while ensuring this position over time.

The closest background documents are European patent EP2380533 and utility model ES1070884, which have an arch in the posterior part of the ferrule protruding from the region of the molars at a downwards angle so that this posterior arch prevents the displacement of the tongue. However, in both inventions, the arch protrudes from the posterior part of the ferrule, which keeps the arch from having some flexibility and when swallowing saliva, this stiffness is very annoying for the user who rejects it or even, due to the force made by the tongue when swallowing saliva, the arch may break or the ferrule may become dislodged.

The aim of this novel intraoral ferrule with arch for preventing snoring and apnea is to prevent these drawbacks by giving the arch some flexibility since the arch protrudes from the inner and anterior part of the ferrule and goes parallel to the bite plane, separated a few mm from the bite plane so that when the action of swallowing saliva is done, it does not have any friction or interference when making the movement towards the palate in order to facilitate the action of swallowing.

At no time is an intraoral ferrule with an arch for preventing snoring and apnea which starts from the inner anterior region of the ferrule as described in the present invention patent reflected in the state of the art.

### Description of the invention

In order to alleviate or, if appropriate, eliminate all the aforementioned drawbacks, this novel intraoral ferrule is presented with an arch which starts from the anterior inner region of the ferrule to the posterior part and goes parallel to the bite plane separated a few mm therefrom so that when the action of swallowing saliva is done, it does not have any friction or interference when making the movement towards the palate in order to facilitate the action of swallowing.

Due to the distance between the point from which the arch protrudes in the inner anterior part of the ferrule and the inclined posterior part, overall, the arch has a certain flexibility which enables it to offer less resistance to the tongue when swallowing saliva.

The more anterior the arch protrudes, the more distance there is with the posterior inclined region of the arch and the more flexibility and less resistance of the arch on the tongue are achieved when swallowing saliva.

The most important advantages of this novel intraoral ferrule are:
- Lower risk of breakage of the arch due to the flexibility thereof.
- Less rejection of the user by facilitating swallowing saliva by retaining the tongue less.

### Description of the drawings

As a complement to the present description, and for the purpose of helping to make the features of the invention more readily understandable, said description is accompanied by a series of figures constituting an integral part of the same, which by way of illustration and not limitation represent the following:

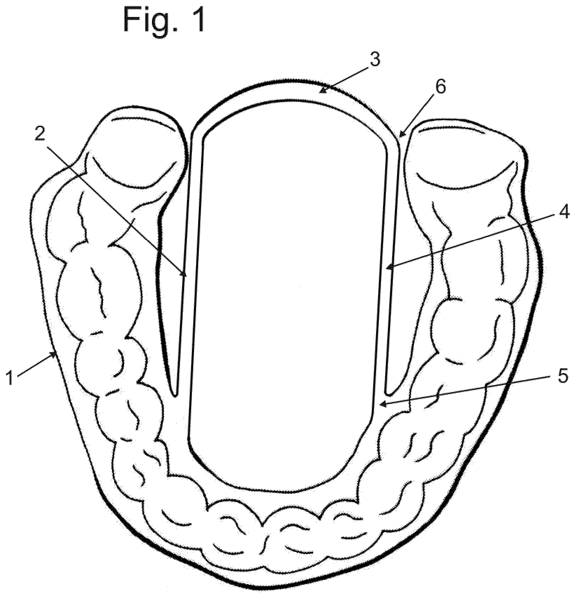

Figure 1 shows a top view of the intraoral ferrule with arch starting from the anterior region to the posterior region.

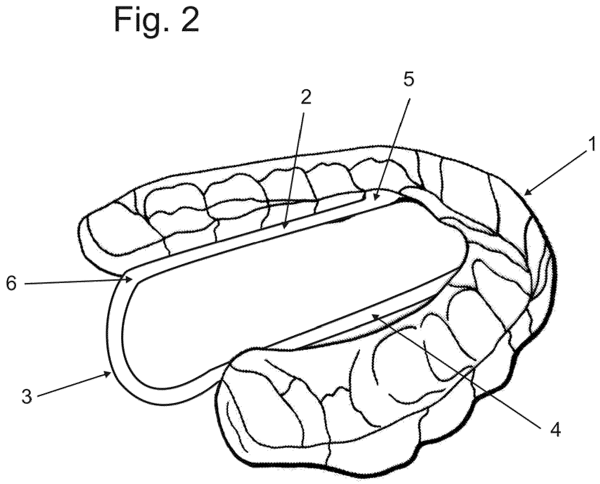

Figure 2 shows a perspective view of the intraoral ferrule with arch for preventing snoring and apnea.
Figure 3 shows a side cross-sectional view of an example of application of the intraoral ferrule with arch for preventing snoring and apnea, with the tongue in the normal resting state thereof.

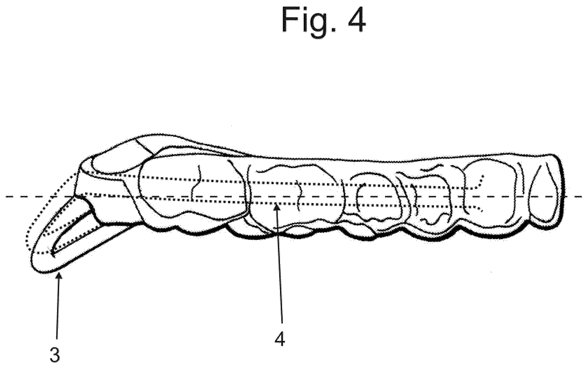

Figure 4 shows a side view of the ferrule showing how the flexibility of the intermediate extensions of the arch enables a vertical displacement of the final curved region of the arch.
Figure 5 shows a top view of the ferrule wherein it shows that the extensions protruding from the anterior inner part have a shape which is not straight.

### Preferred embodiment of the invention

This intraoral ferrule with arch for preventing snoring and apnea is composed of a ferrule (1) which fits into the user's teeth, said ferrule incorporating an arch (2).

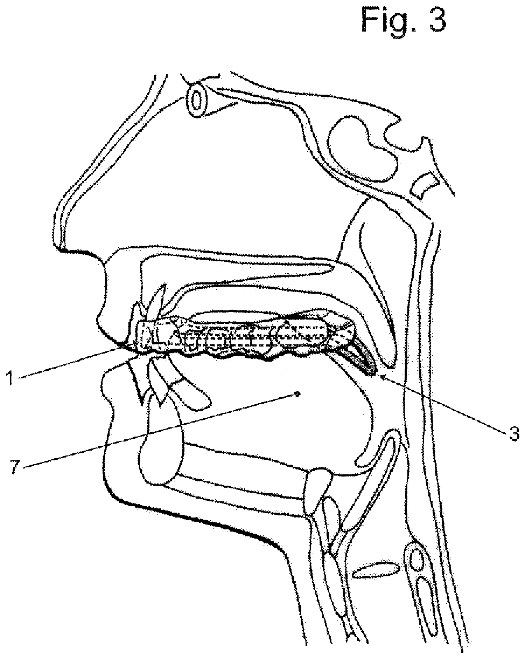

This arch (2) is U-shaped and protrudes from the anterior inner part (5) of the ferrule, at the height of the canines and the extensions (4) of which go parallel to the bite plane (the plane defined between the lower and upper arches when they are in contact with each other), separated a few mm from the plane thereof, to a point (6) located at the rear end of the ferrule, at the bottom of the oral cavity, (see Fig. 3),at which they turn towards the lower part and are joined together by a curved region (3) that is the one which acts by retaining the tongue (7). The extensions may not go parallel to the bite plane and have a maximum inclination of 15° with respect to this plane towards the palate or towards the tongue.

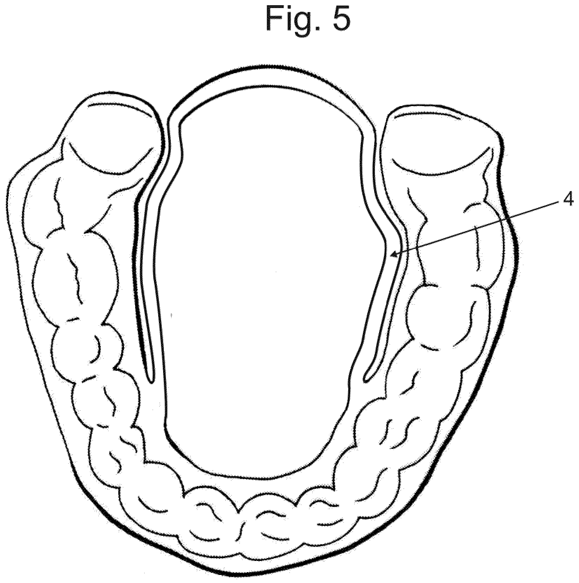

In another preferred embodiment, as seen in Figure 5, the extensions (4) which go parallel to the bite plane, instead of being straight and parallel to each other, can have another varied shape as long as they are parallel to the bite plane and the flexibility thereof enables a displacement of the curved region (3) which forms the end of the arch with respect to the bite plane.

In other preferred embodiments, the arch (2) which protrudes from the anterior inner part (5) does so at the height of the incisors, the canines or the premolars. It must always be taken into account that the further back it protrudes, the less elasticity is conferred to the arch since these extensions are shorter.

Likewise, in other alternative embodiments, the region (3) which forms the end of the arch, and that is the one which acts by retaining the user's tongue (7), has any other geometric shape that does not have to be curved.

Once the nature of the present invention has been sufficiently described, it only remains to add that said invention may undergo certain variations in the composition thereof, as long as said alterations do not substantially vary the features claimed below.

## Claims

1. An intraoral ferrule with an arch for preventing snoring and apnea composed of a ferrule (1) which is adapted to fit onto the user's teeth incorporating an U-shaped arch (2), **characterized in that** the arch (2) starts from the anterior inner part (5) of the ferrule and comprises flexible extensions (4) of which go parallel to the bite plane up to a point (6) at the posterior rear end of the ferrule, at which they turn downwards and are joined together by a region (3) which forms the end of the arch of said 'U' therefore retaining a posterior region of the user's tongue (7) in use.

2. An intraoral ferrule with arch for preventing snoring and apnea, according to the claim 1, **characterized in that** said 'U' arch (2) protrudes from the anterior inner part (5) at the height of the incisors, the canines or the premolars in use.

3. An intraoral ferrule with arch for preventing snoring and apnea, according to the preceding claims, **characterized in that** said region (3) which forms the end of the arch has a curved shape or any other geometric shape.

4. An intraoral ferrule with an arch for preventing snoring and apnea composed of a ferrule (1) which is adapted to fit onto the user's teeth incorporating an 'U'-shaped arch (2), **characterized in that** the arch (2) starts from the anterior internal part (5) of the ferrule and comprises flexible extensions (4) having a maximum inclination with respect to the bite plane of 15° and running up to a point (6) at the rear end of the ferrule, at which they turn downwards and are joined together by an area (3) that forms the end of the arch of said "U", therefore retaining a posterior region of the user's tongue (7) in use.

## Patentansprüche

1. Eine intraorale ferrule mit einem bogen zur verhinderung von schnarchen und apnoe, bestehend aus einer ferrule (1), die an die zähne des benutzers angepasst ist und einen u-förmigen bogen (2) aufweist. Der bogen (2) beginnt am vorderen innenteil (5) der ferrule und weist flexible verlängerungen (4) auf, die parallel zur bissebene bis zu einem punkt (6) am hinteren ende der ferrule verlaufen. Dort biegen sie nach unten ab und sind durch einen bereich (3) miteinander verbunden, der das ende des u-bogens bildet und so bei gebrauch einen hinteren bereich der zunge (7) des benutzers festhält.

2. Eine intraorale ferrule mit bogen zur verhinderung von schnarchen und apnoe gemäß anspruch 1, **dadurch gekennzeichnet, dass** der u-bogen (2) bei gebrauch auf höhe der schneidezähne, eckzähne oder prämolaren aus dem vorderen innenteil (5) herausragt.

3. Eine intraorale ferrule mit bogen zur verhinderung von schnarchen und apnoe gemäß den vorhergehenden ansprüchen, **dadurch gekennzeichnet, dass** der bereich (3), der das ende des bogens bildet, eine gekrümmte oder eine andere geometrische form aufweist.

4. Eine intraorale ferrule mit bogen zur verhinderung von schnarchen und apnoe, bestehend aus einer ferrule (1), die an die zähne des benutzers angepasst ist und einen u-förmigen bogen (2) aufweist. Der bogen (2) beginnt am vorderen innenteil (5) der ferrule und umfasst flexible verlängerungen (4) mit einer maximalen neigung von 15° zur bissebene. Diese verlaufen bis zu einem punkt (6) am hinteren ende der ferrule, an dem sie nach unten abknicken und durch einen bereich (3) miteinander verbunden sind, der das ende des u-bogens bildet und so den hinteren bereich der zunge (7) des benutzers bei gebrauch festhält.

## Revendications

1. Ferrule intra-orale avec arche pour la prévention du ronflement et de l'apnée, composée d'une ferrule (1) adaptée à la dentition de l'utilisateur et intégrant une arche en U (2). Elle est **caractérisée en ce que** l'arche (2) part de la partie antérieure interne (5) de la ferrule et comprend des extensions flexibles (4) parallèles au plan de morsure jusqu'à un point (6) situé à l'extrémité postérieure de la ferrule, où elles s'inclinent vers le bas et sont reliées par une zone (3) formant l'extrémité de l'arche en « U », maintenant ainsi la partie postérieure de la langue (7) de l'utilisateur lors de l'utilisation.

2. Ferrule intra-orale avec arche pour la prévention du ronflement et de l'apnée, selon la revendication 1, **caractérisée en ce que** l'arche en « U » (2) dépasse de la partie antérieure interne (5) à hauteur des incisives, des canines ou des prémolaires lors de l'utilisation.

3. Ferrule intra-orale avec arche pour la prévention du ronflement et de l'apnée, selon les revendications précédentes, **caractérisé en ce que** la zone (3) formant l'extrémité de l'arche présente une forme incurvée ou toute autre forme géométrique.

4. Ferrule intra-orale avec arche pour la prévention du ronflement et de l'apnée, composé d'une embout (1) adaptée à la dentition de l'utilisateur et incorporant une arche en « U » (2). L'arche (2) part de la partie interne antérieure (5) de l'embout et comprend des extensions flexibles (4) présentant une inclinaison maximale de 15° par rapport au plan de morsure et s'étendant jusqu'à un point (6) à l'extrémité arrière de l'embout, où elles se courbent vers le bas et sont reliées par une zone (3) formant l'extrémité de l'arche dudit « U », maintenant ainsi une région postérieure de la langue (7) de l'utilisateur pendant l'utilisation.
